# EUROPEAN PATENT APPLICATION

(11) **EP 4 796 066 A1**
(43) Date of publication of application: **26.08.2026**
(21) Application number: 25315063.5
(22) Date of filing: 25.02.2025
(51) Int. Cl.: A61B 34/30

(54) **CASSETTE FOR USE IN A ROBOTIC DRIVE SYSTEM AND A ROBOTIC DRIVE SYSTEM**

(71) Applicant: Caranx Medical, 75013 Paris (FR)
(72) Inventor: BERTHET-RAYNE, Pierre, 06800 Cagnes-sur-Mer (FR); HANSEN, Jorgen, 06000 Nice (FR); IONITA, Silvia, 06700 Sain Laurent de Var (FR)
(74) Representative: Hepp Wenger Ryffel AG

(57) **Abstract**

A cassette (1) for use in a robotic drive system (2) for driving an elongated medical device (3), in particular a catheter, comprising a housing (4) with a guide path (5) for receiving the elongated medical device (3), at least one pair of drive rollers (61, 62) comprising a first and second drive roller (61, 62) arranged on opposite sides of the guide path (5), wherein the first and second drive roller (61, 62) form at least one driving surface (9) for engaging the elongated medical device (3) between the drive rollers (61, 62) in an operating position, characterized in that the first and second drive roller (61, 62) comprise at least one respective force transfer surface (10) through which they are movably engaged with each other in the operating position to enable force transfer from a driven drive roller (61) to the other of the drive rollers (62).

## Description

The present invention is a cassette for use in a robotic drive system and a robotic drive system according to the preamble of the independent claim. In the field of transcatheter aortic valve implantation (TAVI), an aortic valve of the heart is implanted through the blood vessels without actual removal of the native valve. The valve delivery system is inserted in the body, the valve is positioned and then implanted inside the diseased aortic valve, and then the delivery system is removed.

Manually controlling such a delivery system lacks precision, stability of motion and force interaction, making automation a highly desirable solution.

Assistive surgical robotic systems can be used during interventions such as TAVI. Assistive surgical robotic systems are available to physicians for controlling elongate medical devices such as catheters, endoscopes and other surgical tools within a patient. Such systems comprise a plurality of components that work in tandem to facilitate surgical intervention. The use of so-called cassettes for assistive surgical robotic systems allows for the elongated medical device to be driven during surgery.

There are currently multiple device types in clinical use and multiple approaches known in the prior art to facilitate surgery using an assistive robotic system.

WO 2020/072543 A1 discloses systems and methods for device-assisted manipulation of an implant's position in a patient, such as for delivering and positioning a cochlear implant. An exemplary modular system includes an implant position manipulator (IPM) unit reversibly engaging an elongate member of an implant, and a user control device for applying driving force to the IPM unit to regulate the motion of the implant. The system may include sensors providing feedback on the position or the motion of the implant, or the force or friction applied to the implant during the implantation procedure.

WO 2009/137410 A1 discloses a catheter drive cassette configured to be removably coupled to a motor drive base, comprising a first axial drive mechanism configured to drive a guide wire along its longitudinal axis; a second axial drive mechanism configured to drive a working catheter along its longitudinal axis; and a first rotational drive mechanism configured to rotate the guide wire about its longitudinal axis is described.

US 2021/0330411 A1 discloses a protective envelope for a robotic module for driving a flexible elongate medical element, intended to form a barrier between the robotic driving module and the flexible elongate medical element. The envelope includes a mechanism for attachment to the robotic module, and a guide track for the flexible elongate medical element including a longitudinal axis and a main portion having a free surface of concave shape, the main portion including a longitudinal groove for receiving the flexible elongate medical element, extending along the longitudinal axis and hollowed out in the free surface having a concave shape.

US 2023/000573 A1 discloses a robotic instrument driver for elongate members which includes a first elongate member, and at least one manipulator mechanism configured to manipulate the first elongate member, and at least one articulating drive configured to articulate the first elongate member, positionable on a bed and beside a patient access site. The manipulator and articulating drive are positioned relative to each other a distance less than the insertable length of the first elongate member, stationary in position.

It is an objective of the present invention to overcome the drawbacks of the prior art and provide a solution that is more versatile and accessible, in particular it is an objective to provide a solution for the manipulation of large catheters with sufficient insertion forces, robotic manipulation of instruments without causing damage to them, and a universal design for actuating of a plurality of surgical instruments.

These objectives are solved by a cassette for use in a robotic drive system and a robotic drive system according to the characterizing portion of the independent claim. Preferred embodiments are outlined in the dependent claims.

A cassette for use in a robotic drive system for driving an elongated medical device according to the invention comprises a housing with a guide path for receiving the elongated medical device. At least one pair of drive rollers with a first and second drive roller is arranged on opposite sides of the guide path. The first and second drive roller form at least one driving surface for engaging the elongated medical device between the drive rollers in an operating position.

The first and second drive roller comprise at least one respective force transfer surface through which they are movably engaged with each other in the operating position to enable force transfer from a driven drive roller to the other of the drive rollers.

The cassette can be used with a variety of different elongated medical devices. According to the invention, preferably only one elongated medical device is operated at once. Force is transferred from the driven drive rollers to non-driven drive rollers. The invention allows for direct force transfer from one drive roller to another via the force transfer surfaces resulting in reduced slippage and higher precision. Preferably, the elongated medical device is a catheter. The cassette can be made of plastic, such as nylon, and metal, such as stainless steel.

A form-fit connection can be formed between the force transfer surfaces in the operating position of the cassette. Preferably, the form-fit connection is formed through interacting protrusions and/or interacting recesses, of the transfer surfaces.

This embodiment increases force transfer between the rollers through form-fit resulting in a further reduction of slippage and higher precision.

The cassette preferably comprises at least a measurement roller and a counter surface for engaging the elongated medical device. The drive rollers and the measurement roller are spaced apart along the guide path.

This embodiment allows to measure slippage with the measurement roller. In a preferred embodiment, the measurement roller and the counter surface are formed by a pair of idle rollers. The counter surface ensures force transfer from the elongated medical device on the measurement roller.

Optionally, at least one drive roller of a first pair of drive rollers is movably coupled to at least one drive roller of a second pair of drive rollers in an operating position to enable force transfer between the drive rollers.

Not each drive roller pair needs to be driven in this case. Instead, force can be transferred between the drive roller pairs. The driving surface of the drive rollers, the measurement roller and/or the counter roller may comprise at least one circumferential groove for at least partially receiving the elongated medical device.

A combination of rollers with and rollers without the groove(s) is also conceivable. Such grooves facilitate the engagement of the elongated medical device in an operating position. Furthermore, the elongated medical device can be guided along the guide path. The grooves additionally increase force transfer when engaged with the elongated medical device.

In a preferred embodiment of the invention, the cassette comprises a port holder adapter arranged on or in proximity to a distal opening of the cassette.

The distal opening is hereafter used to refer to the opening of the cassette closer to an end of the elongated instrument that is inserted into the patient during surgery. In contrast, the proximal opening is farther away from the end of the elongated instrument that is inserted into the patient during surgery, along the length direction of the elongated medical instrument. In this embodiment, the port holder adapter allows the use of different port holders ensuring compatibility with a variety of different elongated medical devices such as catheter systems from different suppliers.

Optionally, the cassette comprises at least one port holder which is removably connectable to the port holder adapter and moveable between a release state for releasing a port and a locking state for locking the port in the port holder. The port holder, preferably comprising a pair of jaws, can be configured to receive the port therebetween, or the port holder can comprise a locking unit for reversibly moving the port holder between the release and the locking state. The port holder, preferably comprising a pair of jaws, can also be configured to receive the port therebetween and can comprise a locking unit for reversibly moving the port holder between the release and the locking state.

The port can thus be held in place during operation of the elongated medical device. The pair of jaws allow the port to be fixated from multiple sides. In a preferred embodiment, the locking unit is a cam lever unit. A cam lever unit allows for simple and reversible locking and release of the port holder. The port holder allows the operator to gain access to the patient body for instance by means of an introducer sheath and/or a trocar.

Optionally, the port holder comprises a deformable port-contacting surface.

According to this embodiment, the deformable port-contacting surface is configured for receiving and frictionally engaging at least a portion of the port. The deformable port-contacting surface permits the port some flexure while simultaneously holding it in place.

Optionally, the cassette is convertible between the operating position and a loading position by moving the first and/or second drive or idle roller(s) relative to the guide path. Only one or both rollers can be movable.

The loading position is for loading the elongated medical device. Furthermore, the distance between the first and the second roller of the at least one pair of rollers in the loading position is sufficient to introduce the elongated medical device into the guide channel. In a preferred embodiment, the rollers are mounted on a guide such that they are movable perpendicularly to the guide path.

Optionally, the cassette comprises a carriage. The counter roller and/or the second drive roller is arranged on the carriage.

The rollers on the same side of the guide path are arranged on the carriage. Rollers arranged on the carriage can be moved independently of rollers not arranged on the carriage.

Optionally, the cassette comprises an actuation unit for transitioning the cassette between the operating position and the loading position by moving the carriage.

The actuation unit allows an operator to readily transition the cassette between the operating position and the loading position. In a preferred embodiment, the actuation unit comprises a manual actuation element.

According to a further preferred embodiment, the carriage comprises a guide surface for interacting with the manual actuation element.

The carriage can be biased to automatically assume the loading position, unless the actuation unit is actuated.

In a preferred embodiment, the carriage is spring-biased.

Optionally, the carriage is coupled to at least one guide rail. The guide rail is arranged on the cassette and the carriage is movable along a longitudinal direction of the at least one guide rail.

In a preferred embodiment, the carriage is coupled to two guide rails.

Optionally, one roller is biased toward the opposite roller of the respective pair by means of a biasing element.

This allows for the elongated medical device to be engaged from both sides. Furthermore, the engagement with the force transfer surfaces between rollers can be established and maintained during operation. This reduces slippage and ensures precision of control.

In a preferred embodiment, at least the counter roller is biased toward the opposite roller. It is further preferred that the biasing element is a spring element.

The cassette may further comprise at least one measurement coupling which is connected to at least one of the measurement roller(s) for transferring rotational movement from the measurement roller to an encoder. Alternatively or additionally, it may have at least one drive coupling which is connected to at least one of the drive roller(s) for transferring rotational movement from a drive module to the drive roller.

The drive coupling facilitates the transfer of movement from a drive module that is not part of the cassette to the cassette and the drive roller(s). The measurement coupling allows for the transfer of movement from the cassette to an encoder that is not part of the cassette. Providing an encoder and a drive module not as part of the cassette reduces the number of moving parts and makes the cassette easier to manufacture.

Optionally, the drive coupling is arranged on a connection surface form-fittingly and removably connectable to the drive module.

A form-fitting connection of the drive coupling to the drive module allows for simple installation by mounting. In a preferred embodiment, the cassette comprises a measurement coupling which is arranged on the connection surface. The form-fitting connection of the measurement coupling to the encoder allows for simple installation by mounting. Preferably, the connection surface, which is arranged on the cassette allows for both of the above.

Optionally, the connection surface comprises at least one of a latching element or a securing pin. The connection surface is configured to be convertible by a release element, in particular a release element of the cassette, between a secured position in which the latching element or the securing pin engages a drive module surface immovably and a release position in which the drive module surface is disengaged.

It is further preferred for the drive module surface to comprise a latching element or a securing pin correspondingly shaped and sized to the latching element or the securing pin of the connection surface.

In a preferred embodiment, the latching element is spring-biased.

Optionally, the drive module surface and/or the connection surface comprises alignment elements such as at least one of an alignment recess or an alignment pin which allow for the alignment of the connection surface with the drive module surface.

Optionally, the cassette comprises a cover connected to the housing. The housing is convertible between an open position and a closed position and comprises a sensor which is arranged at least partially on the housing, and the cover, for detecting if the cover is in the open or closed position.

The elongated medical device is protected from external factors by the cover. In addition, the cover can restrain the elongated medical device, keeping it from getting outside of the guide channel. Detecting whether the cover is in the open or closed position ensures proper handling of the cassette. In a preferred embodiment, the cover is hingedly connected to the housing. Further, it is preferred that the sensor comprises a magnet.

Optionally, the cassette can be sealed in a sterile package and configured to be sterilized. Sterilization methods include ethylene oxide sterilization.

The cassette can be single-use or used multiple times. There is no need for the cassette to be sterilized prior to the first use when it comes sealed in the sterile package.

The cassette may comprise a detachable component configured to detach or break off upon intended use of the cassette. This may provide a visual or physical indication that the device has already been used.

A further aspect of the invention relates to a robotic drive system for driving an elongated medical device. The robotic drive system comprises a cassette as outlined above, and a base which is configured for receiving the cassette.

The base comprises the drive module surface including the drive module as described above. This embodiment is modular. The cassette can be exchanged while the base may stay the same. In a preferred embodiment, the base is a robotic arm or part thereof or a separate part mountable on a robotic arm.

Optionally, a foot pedal is be used to operate the elongated medical device via the cassette. The operators' hands remain free.

Optionally, the cassette and the base are configured to be connected in a sealing manner by means of a seal arranged around a base perimeter or by the cassette and the base being shaped and sized to accommodate a sealing drape between the base and cassette.

Optionally, the robotic drive system comprises the encoder for coupling to the measurement coupling and detecting the angular position and/or a control unit for detecting slippage based on the detected angular position and controlling the at least one drive module to counteract the detected slippage. Both are preferably arranged on the base.

If the encoder is arranged on the base, it does not have the size constraints that would apply if it were part of the cassette. The same applies to the control unit. Furthermore, if the cassette does not contain the control unit and the encoder, it consists of less moving parts and does not need to be electrically connected to the base and can be easily disposable. The control unit and the encoder can further be used with different cassettes.

Optionally, the robotic drive system comprises a monitoring unit which is arranged connectably to the cassette to determine whether the cassette is connected to the base, whether the cassette is in the operating position or the loading position, and/or whether the cover is in an open position or a closed position.

In a preferred embodiment, the cassette position is detectable based on the position of the actuation unit.

The rollers are preferably composed of or lined with a compliant material such as silicone, natural rubber, polyisoprene, thermoplastic elastomers and the like. This choice of material enables high force transfer, while preventing damage to the instrument.

The invention is further explained in more detail by means of specific embodiments and the following figures:
Figure 1: A Schematic representation of a possible setup during TAVI including a cassette according to the invention;
Figure 2: A perspective view on the cassette with an open cover in a loading position with a catheter therethrough;
Figure 3: A first cross-sectional view of the cassette in the loading position with the catheter therethrough;
Figure 4: A second perspective view of the cassette according to the invention with parts of a housing removed and in an operating position;
Figure 5: A third perspective view of the cassette in the operating position with the catheter therethrough;
Figure 6: A fourth perspective view of the cassette limited to a distal section with a port holder in a release state and the cover open and the catheter therethrough;
Figure 7: A fifth perspective view of the cassette limited to the distal section with the port holder in a locking state and the cover closed;
Figure 8: A sixth perspective view of the cassette in the loading position with the cover closed;
Figure 9A: A first cross-sectional view of a robotic drive system according to the invention;
Figure 9B: A second cross-sectional view of the drive system limited to a section showing a latching mechanism;
Figure 10: A perspective view of a base as part of the robotic drive system;
Figure 11: A perspective view of the robotic drive system; Unless stated otherwise, like reference numerals are used to refer to the same or similar element.

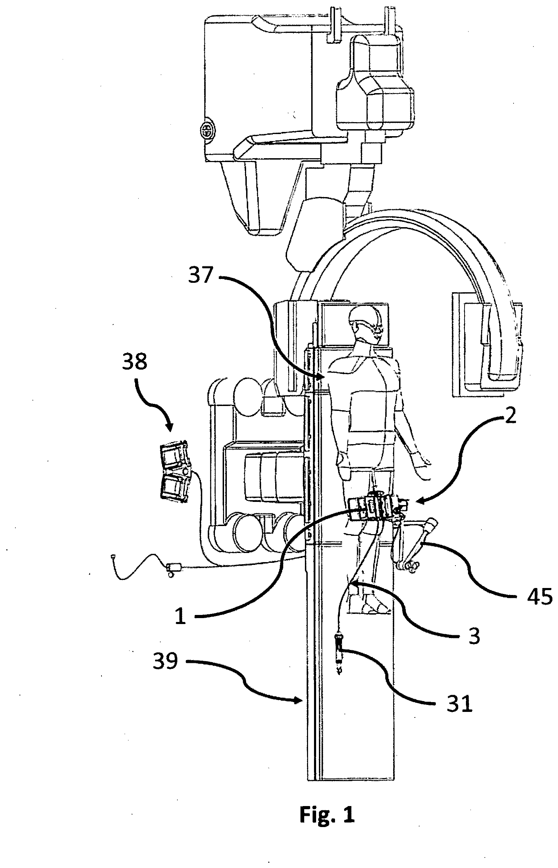

Figure 1 shows a schematic representation of a possible setup during TAVI including a cassette 1 according to the invention. In this embodiment, the cassette 1 is installed as part of a robotic drive system 2. The robotic drive system 2 is arranged on a positioning arm 45 which is attached to an operating table. The cassette 1 drives a catheter 3 during surgical intervention. The catheter 3 is further connected to a catheter delivery system 31. A patient 37 lies on a surgical bed 39. A foot pedal 38 is connected to the robotic drive system 2 in order to control it during the surgery.

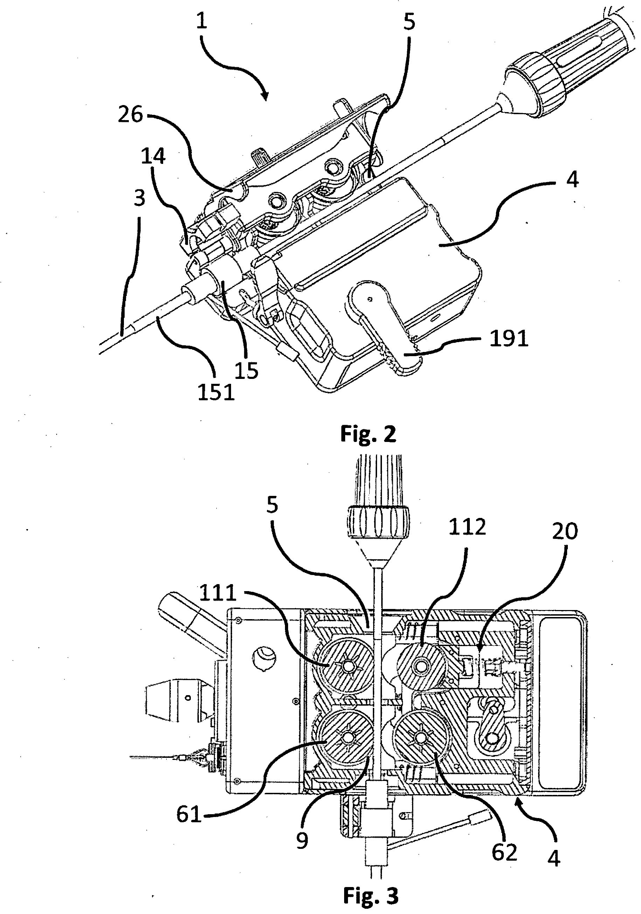

Figure 2 shows a perspective view on the cassette 1 with a cover 26 open in the loading position with the catheter 3 therethrough. The catheter 3 is aligned along a guide path 5 within a housing 4 of the cassette 1. A clamping lever 191 extends away from the guide path 5 in the loading position. An introducer sheath 151 is received in a port 15 which is placed in a port holder 14.

Figure 3 shows a first cross-sectional view of the cassette 1 in the loading position with the catheter 3 therethrough. One pair of drive rollers 61, 62 comprising a first and a second drive roller 61, 62 is arranged on opposite sides of the guide path 5 within the housing 4. A driving surface 9 for engaging the catheter 3 is formed on the drive rollers 61, 62. The driving surface 9 is made from silicone. Proximally to the drive rollers 61, 62, there is a pair of idle rollers 111, 112 spaced apart along the guide path 5. A measurement roller 111 is arranged on the same side of the guide path 5 as a first drive roller 61. A counter roller 112 is arranged on the same side of the guide path 5 as the second drive roller 62. The counter roller 112 is biased toward the measurement roller 111 by a spring 20.

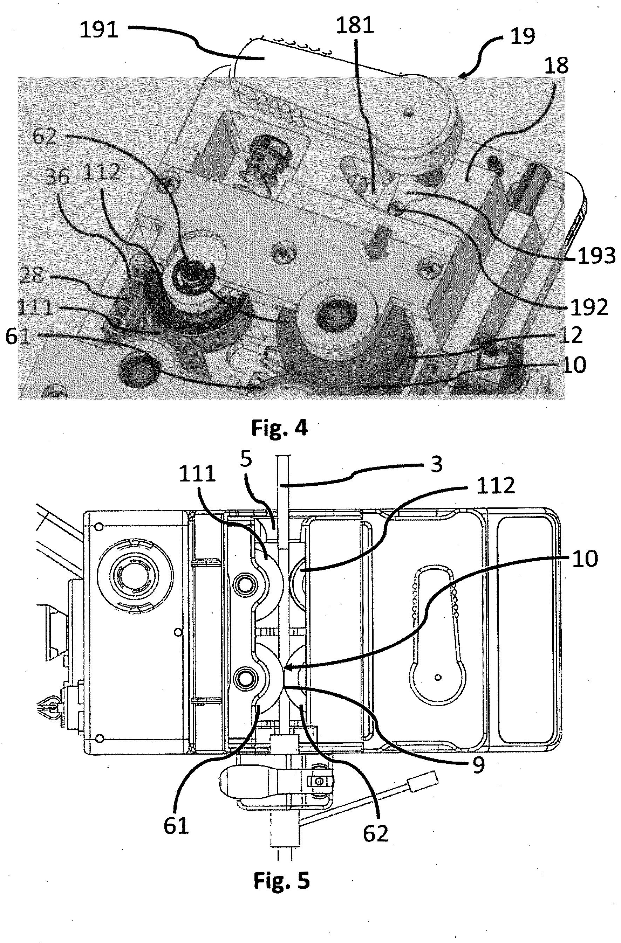

Figure 4 shows a second perspective view of the cassette 1 according to the invention with parts of the housing removed (not shown) and in an operating position. The cassette 1 is not loaded with the catheter 3. A carriage 18 is movably mounted on two guide rails 28. An actuation unit 19 comprises a clamping lever 191 shown in an actuated position. Upon actuation, the clamping lever 191 rotates and an actuation pin 192 arranged on an actuation body 193 interacts with a guide surface 181 of the carriage, forcing the carriage 18 with the counter roller 112 and the second drive roller 62 arranged on it, to move along the guide rails 28 toward the measurement roller 111 and the first drive roller 61 until a predefined compression force between the rollers is achieved in the operating position. Interaction of the actuation pin 192 with the guide surface 181 on the carriage 18 keeps the cassette 1 in the operating position against the bias provided by springs 36. The bias springs 36 are arranged surrounding one rail 28 each. The second drive roller 62 comprises a circumferential groove 12 and is movably engaged with the first drive roller 61 through force transfer surfaces 10 arranged circumferentially to the rollers 61, 62.

Figure 5 shows a third perspective view of the cassette 1 in the operating position with the catheter 3 therethrough. The first 61 and the second 62 of the pair of drive rollers 61, 62 each comprise a groove (not shown) and engage the catheter 3 through the driving surface 9. The pair of idle rollers 111, 112 comprises the measurement roller 111 and the counter roller 112. The measurement roller 111 comprises a groove (not shown) whereas the counter roller 112 does not. The measurement roller 111 and the counter roller 112 both engage the catheter 3. During operation, the driven drive roller 61 is rotated by means of a drive module (not shown). The resulting force is transferred to the second drive roller 62 through the force transfer surface 10 and the catheter 3. As the catheter 3 is moved along the guide path 5, force is transferred from the catheter 3 to the measurement roller 111, which transfers the rotation to an encoder (not shown) to determine slippage. The counter roller 112 ensures that the catheter is firmly engaged with the measurement roller 111 by applying compression force through the biasing spring (not shown). The groove (not shown) facilitates guidance of the catheter 3.

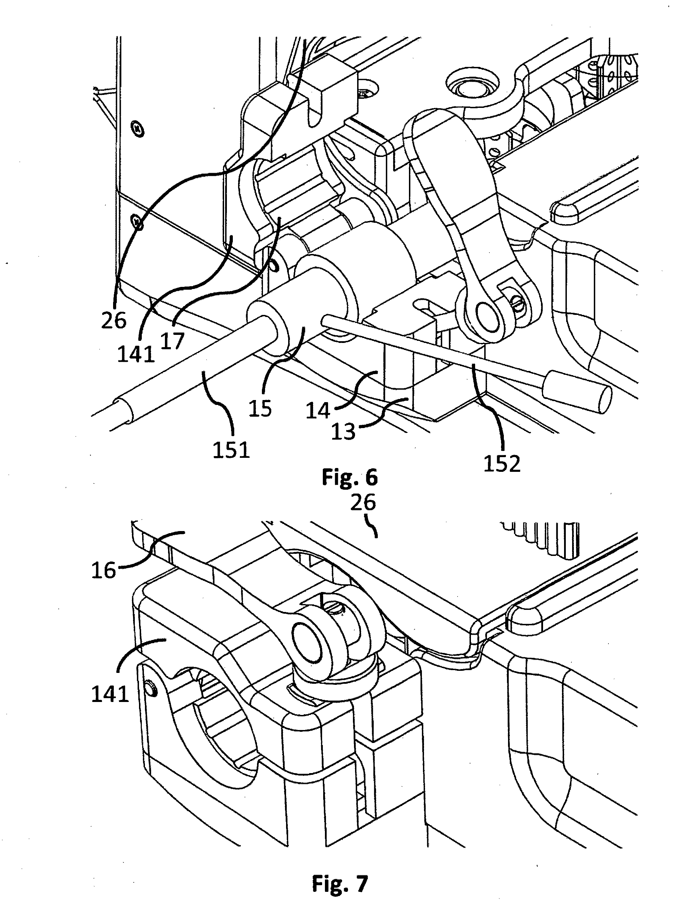

Figure 6 shows a fourth perspective view of limited to the distal section of the cassette 1 with the port holder 14 in a release state and the cover open and the catheter 3 therethrough. The port holder 14 comprises two jaws 141. The upper jaw 141 is open in the release state and is lined with a port-contacting surface 17. The port 15 is engaged to one jaw 141. A port holder adapter 13 is arranged on the distal opening of the cassette 1 and is removably connected to the port holder 14. The introducer sheath 151 is integrally connected to a flush port 152 in the form of a side-arm.

Figure 7 shows a fifth perspective view of the distal section of the cassette 1 with the port holder 14 in a locking state and the cover closed. A cam lever unit 16 is positioned to bring the jaws 141 in engagement with each other.

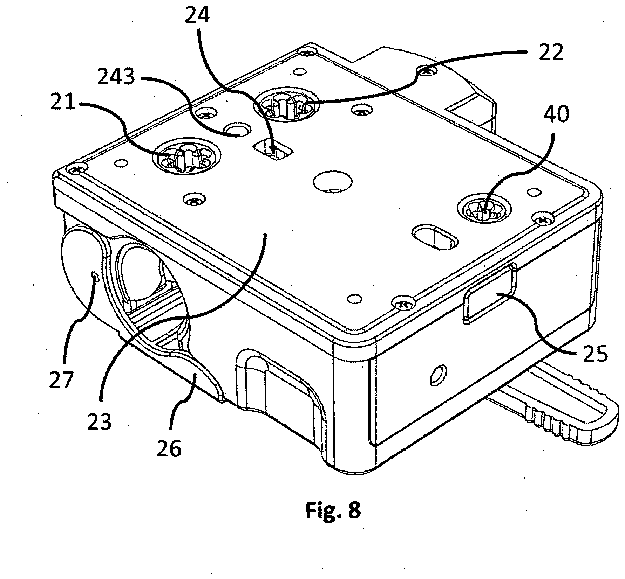

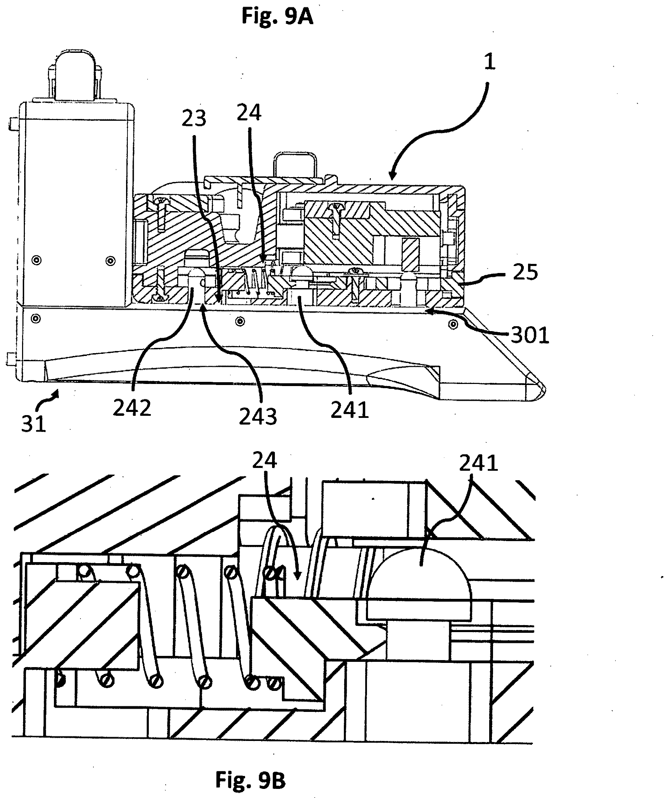

Figure 8 shows a sixth perspective view of the cassette 1 in the loading position with the cover closed. The cover comprises part of a magnet 27 to detect that the cover 26 is in the closed position. A connection surface 23 comprises a drive coupling 22, a measurement coupling 21 and a lever coupling 40. The lever coupling 40 is used to detect the position of the clamping lever 191. The drive coupling 22 and the measurement coupling 21 transmit torque to and from the rollers, respectively. All three couplings 21, 22, 40 are spline-profiled to facilitate force transmission. A release button 25, allows for latching elements 24 that are found in recesses on the connection surface 23 to be moved (see Figure 9A and 9B). Additionally, there are alignment recesses 243 arranged on the connection surface 23 to receive corresponding alignment pins (see Figure 9A).

Figure 9A shows a cross-sectional view of the robotic drive system 2. The cassette 1 is received on a robotic arm 31. A base 31 comprises a drive module surface 301 which comprises securing pins 241 and alignment pins 242. The cassette 1 comprises a latching element 24 that engages the securing pin 241 form- and force-fittingly as seen in Figure 9B by means of a spring mechanism. The alignment pins 242 are arranged on the drive module surface 301 are form-fittingly inserted into alignment recesses 243 on the cassette 1. During mounting, the latching elements 24 on the cassette 1 engage with the securing pins 241 on the base 31, while the alignment pins 242 of the base 31 protrude into the alignment recesses 243 of the cassette 1. Pressing the release button 25 disengages the latching elements 24 from the securing pins 241 allowing the cassette 1 to be unmounted.

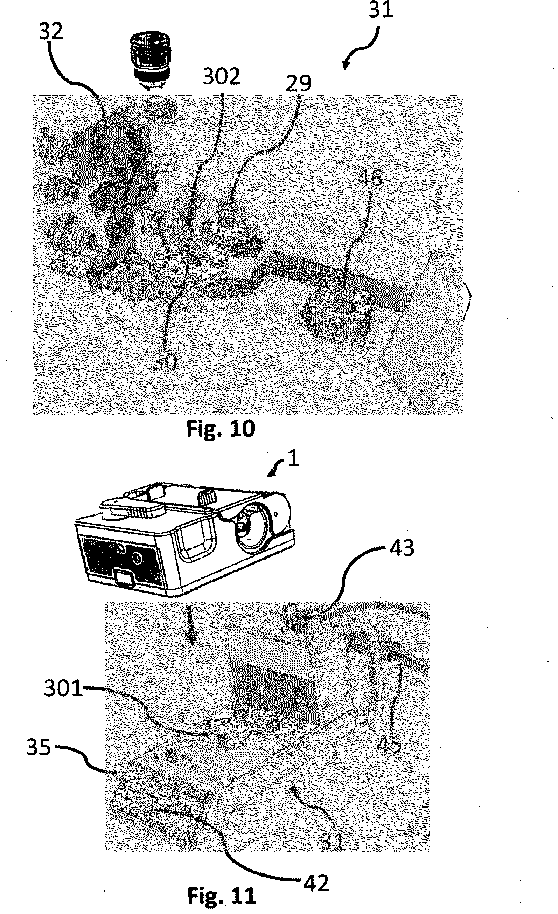

Figure 10 shows a perspective view of the base as part of the robotic drive system 2. Arranged on the base 31 are the encoder 29, the drive module 30, and the lever position detector 46 complementary to the couplings on the connection surface of the cassette (not shown). The encoder 29 is arranged to be coupled with the measurement coupling (not shown) and the drive module 30 is arranged to be coupled with the drive coupling (not shown). The drive module 30 is connected to a control unit 32, comprising a motor, by means of a drive belt 302. The encoder 29 is also connected to the control unit 32, allowing the control unit 32 to adjust the rotation of the driven roller (not shown) based on the measurement roller (not shown) input during operation.

Figure 11 shows a perspective view of the robotic drive system 2. The cassette 1 can be attached to the robotic drive system 2 as indicated, facing the drive module surface 301 on the base 31. The base 31 further comprises an emergency button 43 atop to stop the robotic drive system 2. Control buttons 42 are arranged on the base 31 and facilitate the operation of the robotic drive system 2, particularly the control buttons 42 allow for controlling the catheter 3 once the cassette 1 is mounted and loaded. Cables to and from the base 31 are routed through the positioning arm 45.

## Claims

1. A cassette (1) for use in a robotic drive system (2) for driving an elongated medical device (3), in particular a catheter, comprising:
- A housing (4) with a guide path (5) for receiving the elongated medical device (3),
- At least one pair of drive rollers (61, 62) comprising a first and second drive roller (61, 62) arranged on opposite sides of the guide path (5), wherein the first and second drive roller (61, 62) form at least one driving surface (9) for engaging the elongated medical device (3) between the drive rollers (61, 62) in an operating position,
**characterized in that** the first and second drive roller (61, 62) comprise at least one respective force transfer surface (10) through which they are movably engaged with each other in the operating position to enable force transfer from a driven drive roller (61) to the other of the drive rollers (62).

2. The cassette (1) according to claim 1, wherein a form-fit connection is formed between the force transfer surfaces (10) in the operating position, preferably through at least one of:
- interacting protrusions,
and
- interacting recesses,
of the transfer surfaces (10).

3. The cassette (1) according to one of the preceding claims, wherein the cassette (1) comprises at least a measurement roller (111) and a counter surface (112), preferably at least one pair of idle rollers (111, 112), wherein the first idle roller is a measurement roller (111) and the second idle roller comprises a counter surface (112), preferably is counter roller (112), for engaging the elongated medical device (3), wherein the drive rollers (61, 62) and the measurement roller (111) are spaced apart along the guide path (5).

4. The cassette (1) according to claim 3, wherein at least one drive roller (61, 62) of a first pair of drive rollers (61, 62) is movably coupled to at least one drive roller of a second pair of drive rollers in an operating position to enable force transfer between the drive rollers.

5. The cassette (1) according to one of the preceding claims, wherein the driving surface (9) of at least one of:
- the drive rollers (61, 62),
and
- the measurement roller (111),
and
- the counter roller (112),
comprises at least one circumferential groove (12) for at least partially receiving the elongated medical device (3).

6. The cassette (1) according to one of the preceding claims, comprising a port holder adapter (13) arranged on a distal opening of the cassette (1).

7. The cassette (1) according to claim 6, comprising at least one port holder (14) which is removably connectable to the port holder adapter (13) and moveable between a release state for releasing a port (15) and a locking state for locking the port (15) in the port holder (14), wherein
- the port holder (14), preferably comprising a pair of jaws (141), is configured to receive the port (15) therebetween,
or
- the port holder (14) comprises a locking unit (16), preferably comprising a cam lever unit (16), for reversibly moving the port holder (14) between the release and the locking state,
or
- the port holder (14), preferably comprising a pair of jaws (141), is configured to receive the port (15) therebetween,
and
- the port holder (14) comprises a locking unit (16), preferably comprising a cam lever unit (16), for reversibly moving the port holder (14) between the release and the locking state.

8. The cassette (1) according to claim 7, wherein the port holder (14) comprises a deformable port-contacting surface (17).

9. The cassette (1) according to one of the preceding claims, wherein the cassette (1) is convertible between the operating position and a loading position for loading the elongated medical device (3) by moving, preferably perpendicularly,
- the second drive roller (62) and not the first drive roller (61) relative to
- the guide path (5),
or
- the first drive roller (61) and not the second drive roller (62) relative to
- the guide path (5),
or
- the first drive roller (61) and the second drive roller (62) relative to
- the guide path (5).

10. The cassette (1) according to one of the preceding claims, wherein the cassette (1) comprises a carriage (18), wherein at least one of:
- the counter roller (112),
and
- the second drive roller (62),
is arranged on the carriage (18).

11. The cassette (1) according to claims 9 and 10, wherein the cassette (1) comprises an actuation unit (19) for transitioning the cassette (1) between the operating position and the loading position by moving the carriage (18), in particular with the actuation unit (19) comprising a manual actuation element (191), and preferably a guide surface (181) of the carriage (18) interacting with the manual actuation element (191).

12. The cassette (1) according to claim 11, wherein the carriage (18) is biased to automatically assume the loading position, unless the actuation unit (19) is actuated, wherein the carriage (18) is preferably spring-biased (36).

13. The cassette (1) according to one of the claims 10 to 12, wherein the carriage (18) is coupled to at least one guide rail (28), in particular two guide rails (28), and movable along a longitudinal direction of the at least one guide rail (28).

14. The cassette (1) according to one of the preceding claims, wherein at least one of said rollers (61, 62, 111, 112), in particular at least the counter roller (112) according to claim 3 or 4, is biased toward the opposite roller of the respective pair by means of a biasing element (20), in particular by means of a spring element (20).

15. The cassette (1) according to one of the preceding claims, wherein the cassette (1) comprises at least one of:
- at least one measurement coupling (21) which is connected to the at least one measurement roller (111) for transferring rotational movement from the measurement roller (111) to an encoder (29),
and
- at least one drive coupling (22) which is connected to at least one of the drive roller(s) (61, 62) for transferring rotational movement from a drive module (30) to the drive roller.

16. The cassette (1) according to claim 15, wherein the drive coupling (22) is arranged on a connection surface (23) form-fittingly and removably connectable to the drive module (30), wherein the measurement coupling (21) is preferably arranged on the connection surface (23).

17. The cassette (1) according to claim 16, wherein the connection surface (23) comprises at least one of a latching element (24) or a securing pin (241) and is configured to be convertible by a release element (25), in particular a release element of the cassette (1), between a secured position in which the latching element (24) or the securing pin (241) engages a drive module surface (301) immovably and a release position in which the drive module surface (301) is disengaged, wherein the latching element (24) is preferably spring-biased, and wherein the drive module surface (301) in particular comprises a latching element (24) or a securing pin (241) correspondingly shaped and sized to the latching element (24) or the securing pin (241) of the connection surface (23).

18. The cassette (1) according to one of the preceding claims, wherein the cassette (1) comprises a cover (26) connected to the housing (4), preferably hingedly connected, wherein the housing (4) is convertible between an open position and a closed position, and comprises a sensor (27) which is arranged at least partially on:
- the housing (4),
and
- the cover (26),
for detecting if the cover (26) is in the open or closed position, whereby the sensor (27) preferably comprises a magnet.

19. The cassette (1) according to one of the preceding claims, wherein the cassette (1) is at least one of:
- sealed in a sterile package
and
- configured to be sterilized, in particular by use of ethylene oxide sterilization.

20. A robotic drive system (2) for driving an elongated medical device (3) comprising:
- A cassette (1) according to one of the preceding claims,
- A base (31), in particular a robotic arm, which is configured for receiving the cassette (1),
wherein the base comprises the drive module surface (301) including the drive module (30) according to one of the claims 15 or 17.

21. The robotic drive system (2) according to claim 20, wherein the cassette (1) and the base (31) are configured to be connected in a sealing manner by means of at least one of
- a seal arranged around a base (31) perimeter,
and
- the cassette (1) and the base (31) being shaped and sized to accommodate a sealing drape between the base (31) and cassette (1).

22. The robotic drive system (2) according to claim 20 or 21, wherein:
- the encoder (29) for coupling to the measurement coupling (21) and detecting the angular position,
and
- a control unit (32) for detecting slippage based on the detected angular position and controlling the at least one drive module (30) to counteract the detected slippage,
are arranged on the base (31).

23. The robotic drive system (2) according to one of the claims 20 to 22, wherein the system (2) comprises a monitoring unit which is arranged connectably to the cassette (1) to determine at least one of the following:
- whether the cassette (1) is connected to the base (31),
- whether the cassette (1) is in the operating position or the loading position, wherein the cassette (1) position is detected in particular based on the position of the actuation unit (19),
- whether the cover (26) is in an open position or a closed position.
